(19)

Europäisches
Patentamt
European
Patent Office
Office européen
des brevets

(11) **EP 3 729 086 B1**

(12) **EUROPEAN PATENT SPECIFICATION**

(45) Date of publication and mention
of the grant of the patent:
**20.03.2024  Bulletin 2024/12**

(51) International Patent Classification (IPC):
***G01N 33/52*** *(2006.01)*

(21) Application number: **18830166.7**

(52) Cooperative Patent Classification (CPC):
**G01N 33/52**

(22) Date of filing: **12.12.2018**

(86) International application number:
**PCT/EP2018/084474**

(87) International publication number:
**WO 2019/121198 (27.06.2019 Gazette 2019/26)**

(54) **METHOD FOR BIO-SENSING A BINDING ABILITY AMONG A BIOMOLECULE AND A VIRUS USING VIRAL-LASING DETECTION PROBES**

VERFAHREN ZUM BIOSENSING EINER BINDUNGSFÄHIGKEIT ZWISCHEN EINEM BIOMOLEKÜL UND EINEM VIRUS MITTELS SONDEN ZUR DETEKTION VON VIRUS-LASING

PROCÉDÉ DE BIODÉTECTION D'UNE CAPACITÉ DE LIAISON ENTRE UNE BIOMOLÉCULE ET UN VIRUS À L'AIDE DE SONDES DE DÉTECTION LASER VIRALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

• **MATMON, Guy**
**London NW1 1 BA (GB)**

(74) Representative: **Fischer, Michael**
**Siemens AG**
**Postfach 22 16 34**
**80506 München (DE)**

(30) Priority: **21.12.2017  EP 17209747**
**19.09.2018  EP 18195469**

(43) Date of publication of application:
**28.10.2020  Bulletin 2020/44**

(56) References cited:
**EP-A1- 2 794 861**

(73) Proprietor: **PAUL SCHERRER INSTITUT**
**5232 Villigen PSI (CH)**

(72) Inventors:
• **AEPPLI, Gabriel**
**5210 Windisch (CH)**
• **BAILEY, Joe**
**8005 Zürich (CH)**
• **DALBY, Paul**
**London NW1 9 HG (GB)**
• **HALES, John**
**London SE13 6UJ (GB)**

• **Hales J E: "Implementing the synthetic biology design cycle to fabricate laser dyes", , 31 December 2014 (2014-12-31), XP002787925, Retrieved from the Internet: URL:http://iris.ucl.ac.uk/iris/publication /955819/1 [retrieved on 2019-01-14]**
• **XUDONG FAN ET AL: "The potential of optofluidic biolasers", NATURE METHODS, vol. 11, no. 2, 1 February 2014 (2014-02-01), pages 141-147, XP055541496, New York ISSN: 1548-7091, DOI: 10.1038/nmeth.2805**

**Description**

The present invention relates to a method for biosensing a binding ability among a biomolecule, such as an antibody, a membrane protein or the like, and a virus.

Antibody-based probes tagged for fluorescent quantification can bind a broad range of biomolecular targets with high specificity, but generate a weak signal which can be difficult to distinguish from background noise. Lasing detection probes would be a superior alternative, as they would generate intense, monochromatic signals and the onset of lasing would coincide with a sharp increase in the radiant energy in a resonator-dependent emission line. However, no new detection probes have been developed which can both harness lasing and also bind a broad range of epitopes with the specificity of antibodies, despite the construction of lasers from biological components such as DNA scaffolds and fluorescent-protein-expressing cells, and the synthesis of plasmonic nanolasers. Examples for these kind of analysis have been described in the publications of Hales J E: "Implementing the synthetic biology design cycle to fabricate laser dyes 11 , 31 December 2014 (2014-12-31), XP002787925, Retrieved from the Internet: URL:http://iris.ucl.ac.uk/iris/publication /955819/1 [retrieved on 2019-01-14] and XUDONG FAN ET AL: The potential of optofluidic biolasers, NATURE METHODS, vol. 11, no. 2, 1 February 2014 (2014-02-01), pages 141-147, XP055541496, New York ISSN: 1548-7091, DOI: 10.1038/nmeth.2805 and in the European Patent Application EP 2 794 861 A1

Biolasers, in which biological components form part of the lasing medium, offer greater potential for biocompatibility than plasmonic nanolasers, which are interesting and complex metallic systems. Dye-labelled antibodies themselves are not ideal candidates because they can only typically be conjugated with up to four dyes per antibody before the degree of labelling interferes with target binding and the yield of the dyes, and their size and structure limit the scope for genetic engineering and chemical modification.

By contrast, filamentous bacteriophage M13 - a 7 nm × 900 nm rod-like virus that infects F pilus expressing strains of *E. coli* - has been used effectively as a substitute for antibody probes in cell imaging, flow cytometry and ELISA and as the key component in nanosystems such as virus-based lithium ion batteries and piezoelectric generators. Using phage display, M13 can be routinely programmed to bind specific target biomolecules either by display of known antibody domains or binding proteins fused to either the gene 3 or 8 coat proteins, or by selection from phage-displayed combinatorial libraries to isolate those with the required target-binding specificity and affinity.

It is therefore the objective of the present invention to provide a method for microbiological probes that delivers a significantly higher signal-to-noise ratio in molecular binding assays as compared to the classical dye-based fluorescence probes.

This objective is achieved according to the present invention by a method for biosensing a binding ability among a biomolecule, such as an antibody, a membrane protein or the like, and a virus, comprising the steps of:

a) labelling the virus by conjugating a plurality of dyes, preferably fluorescein dyes, to the virus in order to achieve a labelled virus being controllable with respect to the density of labelled dyes per virus molecule;
b) solutions-phase-wise binding the labelled virus to the biomolecule in a liquid solution to the extent the binding characteristics among the biomolecule and the labelled virus in order to generate biomolecule virus compounds and to allow for a mix-and-measure ligand-binding homogenous assay that non-disruptively measures the lasing response of the biomolecule virus compounds;
c) pumping the liquid solution comprising the biomolecule virus compound with light having a wavelength and/or an intensity being adopted to the type of the effective mechanism of the dyes, preferably the effective fluorescence mechanism of the fluorescein dyes, labelled to the virus;
d) amplifying the light emitted from the pumped dyes in an optical resonator in the attempt to achieve a lasing response from the excited dyes in the optical resonator; and
e) measuring the intensity of the amplified emitted light and evaluating the intensity of the amplified emitted light against the intensity of the amplified emitted light from a known density of labelled fluorescein dyes per virus molecule or against an equivalent concentration of unbound labelled virus with the same number of dyes per virus in order to determine the binding ability of the biomolecule to the labelled virus.

This method therefore represents a viral-lasing detection mechanism which can bind the full range of biomolecules targeted in clinical assays, including proteins, nucleic acids and cells, whilst also generating a lasing signal in an optical configuration compatible with ordinary fluorimeters. The structural order and repeating chemical landscape on the surface of viruses provide a versatile and amenable model system for modulating the spectrum and threshold dynamics of the laser using the principles of synthetic biology. At the transition to lasing, the photon flux from the conjugated viruses increases by five orders of magnitude, the spectral linewidth narrows to below 5.0 nm, and the sensitivity of the output to small changes in the probe concentration or environment is heightened.

Preferred embodiments of the present invention are listed hereinafter and can be applied alone or in arbitrary combinations:

a) the virus is a M13 bacteriophage or a recombinant Tobacco mosaic virus-like particle (rTMV);

b) the biomolecule is an antibody or a monoclonal antibody, preferably an IgG2a monoclonal antibody (mAB);

c) the fluorescein dye is a fluorescein isothiocyanate isomer 1 dye;

d) the virus is covalently modified with the fluorescein dye;

e) the pumping is achieved using 1 ns to 20 ns excitation pulses at a wavelength that is within the visible spectrum, preferably in the range of 450 nm to 600 nm;

f) the liquid solution is circulated between a reservoir and a flow cuvette;

g) the virus is effectively genetically-programmable with a spectral peak emission that is tunable by varying the number of fluorescein dyes attached per virus and/or by modifying the chemical landscape of the surface of the virus; and/or

h) the virus is conjugated with a number of fluorescein dyes equivalent to a range of 0.5 to 2 dyes, preferably 0.7 to 1.2 dyes, on average per ring of coat proteins of the virus.

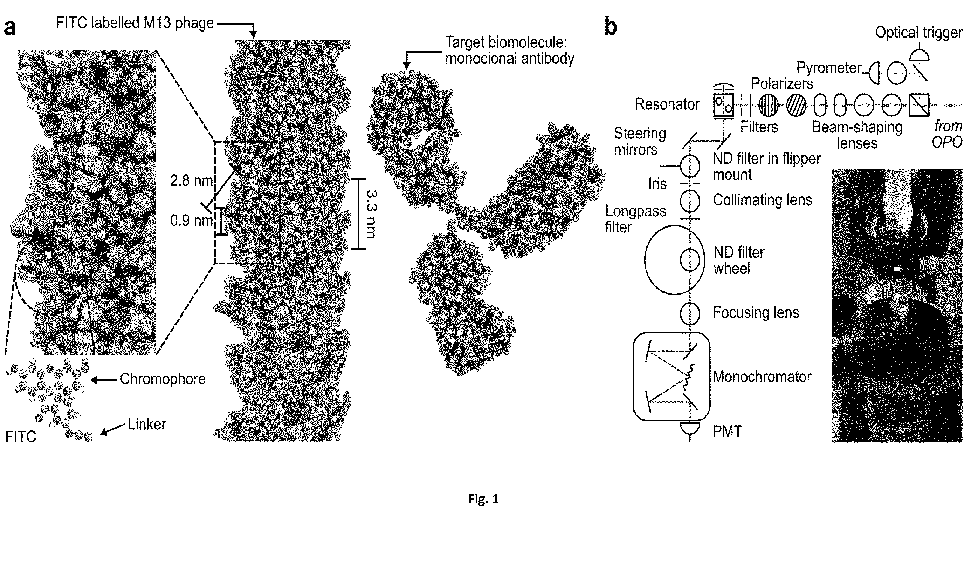

In particular, the surface of the M13 bacteriophage can be functionalised with amine-reactive dyes that could attach to either the $\alpha$-amines at Ala1 or the $\varepsilon$-amines at Lys8 on the 2700 50 amino-acid alpha-helical gene 8 coat proteins or the solvent-exposed primary amines on the five gene 3 coat proteins. The gene 8 coat proteins form an overlapping quasi-crystalline lattice with a 5-fold rotation axis and a two-fold screw axis, which brings the dyes into close proximity - much smaller than the wavelength of visible light - enabling electronic interactions and leading to resonant energy transfer between dye molecules as well as fluorescence quenching (see Figure 1a).

Other advantageous features of the present invention are listed in the depending claims.

Preferred embodiments of the present invention are hereinafter described in more detail with respect to the attached drawings which depict in:

Figure 1    schematically the design of lasing detection probe composed of M13 bacteriophage (PDB: 2MJZ) covalently modified with fluorescein isothiocyanate isomer 1 dyes;

Figure 2    a series of threshold curves for different probe concentrations;

Figure 3    coupling viral-lasing to interactions between neighbouring dyes and their environment;

Figure 4    the response of the viral laser to ligand-binding;

Figure 5    step-changes in the threshold point in response to ligand-binding; and

Figure 6    schematically an optical configuration for experiments conducted using a, resonant cavity R1 and b, resonant cavity R2.

As one representative example, the present invention provides a viral laser from fluorescein-dye-labelled M13 and demonstrates its capabilities as a new analytical platform for biomedicine. Viral-lasing detection probes are the first probes which can bind the full range of biomolecules targeted in clinical assays, including proteins, nucleic acids and cells, whilst also generating a lasing signal in an optical configuration compatible with ordinary fluorimeters. The structural order and repeating chemical landscape on the surface of M13 provide a versatile and amenable model system for modulating the spectrum and threshold dynamics of the laser using the principles of synthetic biology. At the transition to lasing, the photon flux from the probes increases by five orders of magnitude, the spectral linewidth narrows to below 5.0 nm, and the sensitivity of the output to small changes in the probe concentration or environment is heightened. It is shown that a 50 % increase in the concentration of the probes from 100 pmol/mL results in a > 1,000,000 % increase in signal, which is the greatest responsivity to probe concentration shown in any biological assay to the best of our knowledge.

In a proof-of-concept study, a mix-and-measure ligand-binding assay sensitive to 90 fmol/mL monoclonal antibody has been optically engineered, suggesting that clinically-relevant concentrations of biomolecules can be detected without immobilisation of the ligand or probe on surfaces and without invasive wash steps.

Figure 1 shows schematically the concept of viral lasers. Part a) shows a model of the atomic structure of a lasing detection probe composed of M13 bacteriophage (PDB: 2MJZ) covalently modified with fluorescein isothiocyanate isomer 1 dyes (PubChem CID: 18730). There are approximately 540 rings of gene 8 coat proteins per M13, but only 13 are shown here. The target biomolecule is a IgG2a monoclonal antibody (mAb) that can be bound by the gene 3 coat proteins and is represented in the figure by the structure of an intact IgG2a mAb (PDB: 1IGT) which should have a homologous structure. Inset, a zoomed image of the surface of M13 shows the close proximity of the attached dyes. Further inset, the chemical structure of fluorescein isothiocyanate is depicted. Part b) illustrates schematically the optical setup of the viral laser used to construct a mix-and-measure ligand-binding assay. Inset, a photograph of the viral laser emission as viewed from along the axis of the resonator is shown.

For the design of the viral lasing, the properties of viral lasers have been investigated in which the gain medium is a solution of fluorescein-dye-labelled M13 in two contrasting resonator geometries. Resonant cavity R1 was constructed to explore viral-lasing for metrology, and resonant cavity R2 was engineered to strengthen the coupling of the viral-lasing to the near-field and electronic interactions of the dyes (as shown in Figure 1b). In both instruments the excitation and detection optics were similar to those in a fluorimeter, except for the greater than eight-decade dynamic range of the spectrometer and the intensity of the 3 ns to 5 ns excitation pulses at 493 nm (see Figure 1b, details are described in the section Methods hereinafter). The gain medium was circulated between a reservoir and a flow cuvette and the pump light source operated in single-shot mode to allow the dye to be refreshed between pulses as shown in Figure 1b.

The resonator R1 comprises a flat mirror separated by 300 mm from a spherical mirror with a radius of curvature of 400 mm, equivalent to 2 round-trips per FWHM (Full Width at Half Maximum) of the temporal profile of the pump laser pulse. The diameter of the beam was constrained only by the 2 mm $\times$ 2 mm windows of the cuvette. In contrast to microresonators, these dimensions are much greater than microbiological length scales ensuring that there was no mechanical interference from the resonator.

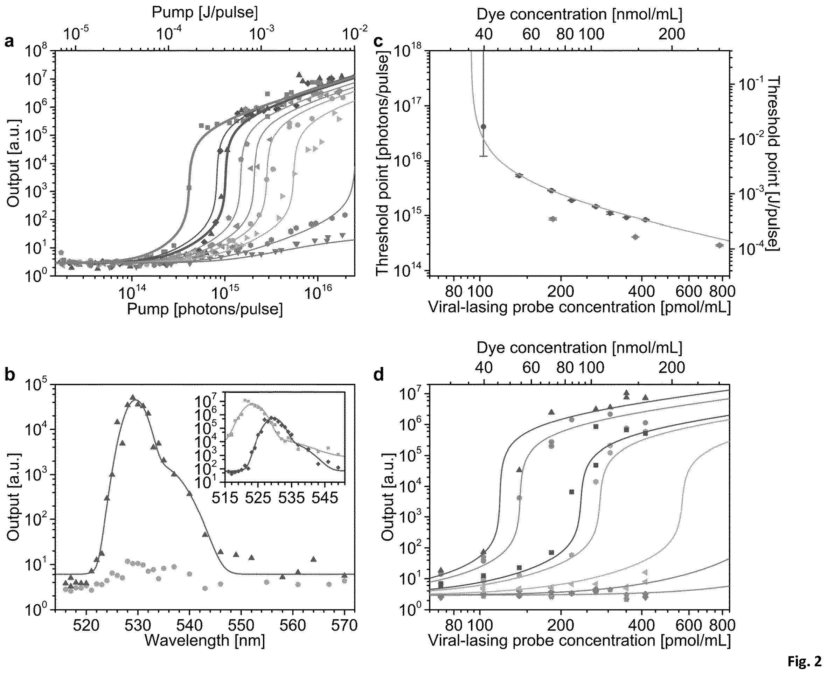

Figure 2 shows lasing action for the functionalised virus and demonstrates its strong sensitivity to the probe concentration in the buffer solution. In detail, Figure 2 shows viral lasers and quantification of viral-lasing probes. (A) Threshold behaviour in R1 for 146 nmol/mL fluorescein (F1, dark orange) and for viral-lasing probes (V1) diluted from 413 pmol/mL (purple) to 351 pmol/mL (dark blue, equivalent to 135 nmol/mL fluorescein), 270 pmol/mL (green), 219 pmol/mL (light purple), 185 pmol/mL (cyan), 140 pmol/mL (light green), 103 pmol/mL (blue), and 72 pmol/mL (grey). The fitted curves represent a global fit of all of the sets of threshold data to a theoretical model. (B) Emission spectra of V1 at 413 pmol/mL as pumping increased from $5.5 \times 10^{14}$ photons/pulse $\pm$ 10.1 % (cyan) to $9.1 \times 10^{14}$ photons/pulse $\pm$ 6.6 % (dark blue). Inset, bathochromic shift in the spectrum of V1 (purple, pumping of $1.5 \times 10^{15}$ photons/pulse $\pm$ 10.9 %) relative to 72 nmol/mL fluorescein (orange, $1.7 \times 10^{16}$ photons/pulse $\pm$ 11.4 %). Curves are fits to the sum of two Gaussian peak functions. The output was not calibrated but was proportional to the number of photons entering the spectrometer (see Methods). (C) Response of the threshold point to variations in probe concentration. Scatter points represent the threshold point calculated by fitting individual sets of threshold data of either V1 (dark blue) or fluorescein (dark orange) using a simple function. The line is the expected dependence of the threshold point on the probe concentration calculated from the global fit in (A). (D) The output at different probe concentrations has been plotted at pump energies of $1.0 \times 10^{16}$ photons/pulse $\pm$ 5.7 % (dark blue), $5.4 \times 10^{15}$ photons/pulse $\pm$ 7.6 % (green), $1.8 \times 10^{15}$ photons/pulse $\pm$ 6.2 % (purple), $1.4 \times 10^{15}$ photons/pulse $\pm$ 5.3 % (cyan), $5.5 \times 10^{14}$ photons/pulse $\pm$ 4.8 % (light green), $2.5 \times 10^{14}$ photons/pulse $\pm$ 6.3 % (blue), $9.6 \times 10^{13}$ photons/pulse $\pm$ 5.8 % (light purple). The lines are simulations from the same global fit (see a) for the output against probe concentration at the same pump energies. In Figure 2a, a series of threshold curves demonstrating laser action for different probe concentrations is displayed as well as for fluorescein in solution with no virus. For the highest probe concentration (purple), 413 pmol/mL M13 conjugated with 386 dyes per M13 (V1) - equivalent to 0.7 dyes on average per ring of proteins - increasing the pump energy by a factor of 2.0 from below to above the threshold point for lasing, increased the output energy at the spectral peak by a factor of 16,000 (Figure 2a).

The threshold region of the viral laser has been measured with unprecedented precision across 7 decades of output because it is this non-linearity that makes lasing such a unique analytical prospect. This contrasts attempts at similar measurements for other biolasers, which have typically been restricted to 1 to 2 decades of dynamic range. Moreover, often only the above-threshold region is measured, which is a region where the output increases linearly with pumping, so the position of the threshold point can only be found from linear extrapolation, reducing the potential utility of these biolasers as analytical instruments. In Figure 2a, the initial deviations in output from sub-threshold behaviour are due to the amplification of seed fluorescence before the gain can overcome the resonator losses, and the inflection points are the transitions to lasing.

The threshold for 146 nmol/mL fluorescein (F1, dark orange) was 2.2 times lower than for 351 pmol/mL V1 (dark blue), which had a similar volume-averaged optical density equivalent to 135 nmol/mL fluorescein. This implies that there was enhanced quenching of the dyes attached to M13 due to dye-dye or dye-protein interactions, since the lasing threshold $\varphi_{th}$ varies according to,

$$(1)\ \varphi_{th} = \frac{K_2\ K_L}{K_F\ (D\ K_{RAD} - K_L)}$$

This equation is derived in the Theoretical models section. The microenvironments provided by the virus and solvent for the dyes will affect the decay rate $K_2$ from the upper state of the dye, but optical absorption data show no influence on the strength $K_F$ of the coupling of the pump to the dyes. If the resonator, defined by the loss rate $K_L$, the rate $K_{RAD}$ of spontaneous emission per dye into the resonator mode and the number $D$ of dyes in the mode volume are unchanged and $D$ is large, the ratio of ultimate lasing output $n$ to pump power $\varphi$ will be the same for V1 and F1 because in the high power limit,

$$(2)\ \frac{n}{\varphi} \rightarrow \frac{\beta\ K_F}{K_{RAD}} \left( \frac{D\ K_{RAD}}{K_L} - 1 \right) \rightarrow \frac{\beta\ K_F\ D}{K_L}$$

where $\beta$ is the optical attenuation due to the output coupler and the spectral bandwidth of the spectrometer (equations derived in Theoretical Models). The data in Figure 2a are consistent with eq. (2).

The curves in Figure 2a have been derived from a model of the threshold dynamics that describes the energy build-up in the dominant mode in terms of the pump energy and the dye concentration while accounting for the seed fluorescence. The data has been globally fit with shared parameters for experimental variables retained between measurements, such as the number of resonator modes, and fixed values for variables which could be calculated in advance, including the rate of loss from the resonator. From this model and by noting that from eq. (1) the threshold point is proportional to ($\left( \frac{D}{D_{min}} - 1 \right)^{-1}$ where $D_{min} = \frac{K_L}{K_{RAD}}$, it has been estimated that the minimum number of probes required for lasing is 92.6 pmol/mL $\pm$ 0.9 pmol/mL, which is approximately three orders of magnitude lower than the lowest reported DNA FRET laser probe concentration. This value is at the upper limit of the useful range for probes in a clinical context, but it could be reduced straightforwardly by decreasing $K_L$ and/or increasing $K_{RAD}$ via steps such as minimising Fresnel reflections at the cuvette side-walls, or by reducing the number of cavity modes with mode selective optical elements or by shortening the resonant cavity.

Spectral linewidth narrowing has not been shown for a solution-state biolaser previously and even here the spectral measurement below threshold has been hampered by low light levels. Even so, Figure 2b demonstrates that the sharp rise in output at threshold coincided with a change in the spectral line-shape and a narrowing of the spectral linewidth to 4.0 nm $\pm$ 0.3 nm (FWHM) above threshold at a pump energy of $9.1 \times 10^{14}$ photons/pulse $\pm$ 6.6 %.

Unlike for a DNA FRET laser or a GFP cell laser, the biological structural order of M13 impacts the spectroscopic characteristics of a homogeneous ensemble of chromophores in the viral laser. Moving the dyes closer together first by increasing the density in solution and then via immobilization on the viruses red-shifted the spectral peak from 523.4 nm $\pm$ 0.2 nm for 72 nmol/mL fluorescein (mean separation 28.5 nm) to 527.2 nm $\pm$ 0.4 nm for 301 nmol/mL fluorescein (17.7 nm) to 529.6 nm $\pm$ 0.2 nm for V1 (Figure 2b inset). Thus, lasing viruses are analogous to genetically-programmable quantum dots whose spectral peak emission can be tuned not by varying their size but by varying the number of dyes attached per virus, or by modifying the chemical landscape of the surface of the virus.

Further, the quantification of viral-lasing probes will be discussed. Figure 2c and Figure 2d show that the concentration of dye-labelled M13 probes can be determined from the position of the threshold point. A simple function has been derived that can model threshold data without requiring precise knowledge of the experimental variables, and in Figure 2c this function has been implemented to calculate the threshold points as a function of the probe concentration (dark blue), and in matching units for freely-dissolved fluorescein (dark orange). The data points are in excellent agreement with the cyan curve, which is the expected threshold point position based on the global model of the threshold dynamics (see Figure 2a), proving that each set of threshold data contained enough information to allow the probe concentration to be calculated precisely.

Typically, threshold data are plotted as the output against pump energy at constant dye concentration, as is the case in Figure 2a, but in Figure 2d it is shown that the same data can be plotted as the output against probe concentration at constant pump energy. Mirroring a conventional threshold curve, the output was extremely sensitive to small changes in the probe concentration when the pump energy was close to the associated threshold point. For instance, from the model globally fit to the data, if the pump energy were fixed at $1.001 \times 10^{16}$ photons/pulse, which was the threshold point for 118.8 pmol/mL V1, a 50 % increase in the probe concentration from 100.0 pmol/mL to 150.0 pmol/mL would result in a 1,100,000 % increase in the output. Away from the threshold point, a 50 % change would simply result in a ~ 50 % change in the output, as is typical for a ratiometric assay, which might be difficult to see above the background. This is the greatest responsivity in output to small changes in probe concentration that has been recorded in any biological assay system, to the best of our knowledge. This analytical concept has immediate applications in tipping point measurements, where the sample would be pumped at the threshold intensity for the critical concentration of lasing probes to yield an unambiguous, digital readout: if there is lasing, then the critical concentration is exceeded, otherwise there is no lasing. Alternatively, the pump energy could be scanned to find the threshold point, and therefore the probe concentration, via cross-referencing with a calibration plot such as Figure 2c.

As for other optical assays, control measurements prior to the addition of the lasing detection probes can fix the contribution of noise sources and any other chromophores in the sample. Beyond this, the potential for multiplexing in viral lasers is intrinsically greater than for fluorescence-based techniques such as flow cytometry because the emission spectral linewidths are narrower, enabling the implementation of companion probes to monitor changes in environmental factors for even greater measurement precision and to make differential measurements possible. To do this, we propose decomposing the overlapping gain spectra of the different probes by sweeping the dispersive element in a tunable viral laser to record the output at different emission wavelengths. Future work will aim to quantify the multiplexing capability of viral lasers, determine the impact of cellular milieu and blood serum on lasing, and harness synthetic biology to engineer environmentally insensitive probes.

Now, coupling viral-lasing with the near-field and electronic interactions of the dyes is discussed. Binding events are likely to disrupt the near-field and electronic interactions occurring at sub-1 ns timescales on the surface of the phage between dyes and between the dyes and their environment. Accordingly, we increased the impact of near-field and electronic interactions occurring at sub-1ns timescales on the surface of the phage by reducing the resonator (designated R2) length to 26 mm, corresponding to a round-trip time of 195 ps taking the refractive index of the gain medium into account (Figure 1b). We increased the effect of binding on a single probe by increasing the density of dye molecules per probe, and reduced the concentration of probes. The number of spatial modes was constrained by introducing a 300 $\mu$m pinhole so that the Fresnel number of the resonator was F = 6.7.

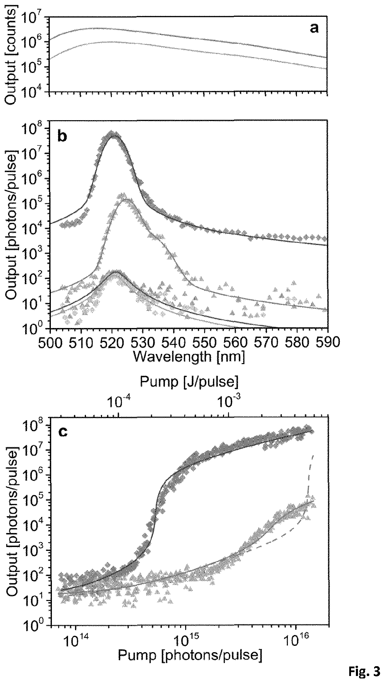

Figure 3 shows the experimental results for 23 pmol/mL M13 conjugated with 564 dyes per M13 (V2), equivalent to 1.0 dye on average per ring of coat proteins, and for 13 nmol/mL unbound fluorescein dye molecules in buffer (F2). Figure 3a shows that the fluorescence yield was broad for both V2 and F2, and that V2 (cyan) was shifted by 4 nm to longer wavelengths, and quenched by a factor of 3.5 per dye relative to F2 (dark orange). In the resonant cavity R2, for pump intensities close to but below the threshold for lasing, the emission spectra for both V2 (light purple) and F2 (light orange) were sharpened considerably to peaks very close to each other at 521.4 nm $\pm$ 0.1 nm and 521.0 nm $\pm$ 0.1 nm, with FWHMs of 7.2 nm $\pm$ 0.3 nm and 6.7 nm $\pm$ 0.3 nm, respectively (Figure 3a, b). This shows that, even below threshold, the interaction between the dyes and the resonant cavity modes is strong enough to narrow the emission peaks compared to free-space fluorescence emission.

As for the more concentrated dye solutions in R1, the transition to lasing led to an increase by many orders of magnitude in the emission intensity at maximum pumping. Above threshold, the V2 emission spectrum (cyan) shows oscillation build-up in two modes - at 524.8 nm $\pm$ 0.16 nm and 530.9 nm $\pm$ 1.4 nm - similar to V1, and compares to just one mode for F2 (dark orange) at 521.1 nm $\pm$ 0.06 nm (Figure 3b). Therefore, above-threshold pumping caused a red-shift in the V2 emission spectrum which was not observed for F2. The primary V2 peak at 524.8 nm was linewidth-narrowed to 5.7 nm $\pm$ 0.22 nm, which was similar to the FWHM of 5.6 nm $\pm$ 0.08 nm for the single F2 peak. The secondary V2 peak had the same Voigt profile as the primary peak but was broader, with a FWHM of 9.6 nm $\pm$ 0.6 nm.

The threshold curves displayed in Figure 3c revealed other differences between V2 (cyan) and F2 (dark orange). Firstly, the lasing threshold of V2 was increased by a factor of 11.1, which was larger than for V1 relative to F1 because of greater non-radiative losses due to the proximity of the dyes, and was consistent with the reduction in fluorescence yield and eq. (1). Secondly, the output above threshold was severely diminished and the approach to saturation was considerably broadened for V2 compared to F2, which was a qualitatively different outcome than for V1 and F1.

The second observation cannot be readily understood in terms of the rate equations used to model the threshold dynamics of F1 and V1 in R1. The parameters $K_F$, $K_{RAD}$, $K_L$, and D were the same for V2 and F2, but, even if they were to vary for V2 to account for the diminished gain above threshold, this should be reflected in a proportionally large increase in the threshold point, which was not observed.

The threshold behaviour of V2 was consistent with additional relaxation channels opening due to the laser emission above the threshold for lasing. Such channels can arise because of reversible photochemistry at the peak emission wavelength between excited state dyes and oxygen, chemical moieties on the surface of M13 or other dyes. Alternatively, such channels may arise due to self-absorption of the stimulated emission by the dyes conjugated to the virus. In the fundamental rate equations 7 and 8 which describe lasing, the same stimulated emission term $\mathbf{K_{RAD}}\mathit{n}\mathbf{D_2}$ both feeds the increase in mode occupancy and depletes the population of dyes in the upper state. The additional relaxation channels would break this symmetry by increasing the rate of upper state decay by a factor of $\xi$, which would renormalize the ratio of lasing output to pump energy in the high power limit described by eq. (2) by a factor of $\mathbf{1}/\xi$ without affecting the position of the threshold point itself. In the model globally fit to the threshold curves, an asymmetry factor of $\xi$ **= 360 $\pm$ 37** for V2 accounts for the flattened response of the output to increased pumping through the threshold point region and the diminished output above threshold, confirming the validity of this theoretical picture (Figure 3c, dashed line is a model fit with $\xi = 1$, for comparison). This effect would be enhanced in R2 compared to R1 because the length of the resonant cavity was shortened without changing the length of the gain medium, so the attenuation of the transmission due to absorption channels on the surface of the virus increased by a power of 10.4 compared to R1, which is the ratio of their respective round-trip times.

In the following, the proof of concept mix-and-measure ligand-binding assay is discussed. Most assay formats gain sensitivity by trading a degree of disruption to the biological system, for instance, by immobilising the ligands or probes and introducing wash steps, or by labelling the ligands. Given the unprecedented signal-generation capabilities of viral-lasing probes, it was intended to test the feasibility of a mix-and-measure ligand-binding assay which does not necessitate these trade-offs. Analogous to a plasmonic approach where ligand-binding to antibodies immobilized on a metal surface modulates the optical response, here the aim was to measure shifts in the threshold point and variations in the output intensity in response to solution-phase binding of the lasing probes. For such an assay, the viral laser needs to be sensitive to the binding of the probes to a target ligand.

Mix-and-measure ligand-binding assays that are minimally disruptive to the biological system under scrutiny would be useful for biological monitoring applications, for instance, in environmental sensing, continuous manufacturing of biopharmaceuticals in industrial bioprocesses and continuous observation of patients in clinical settings.

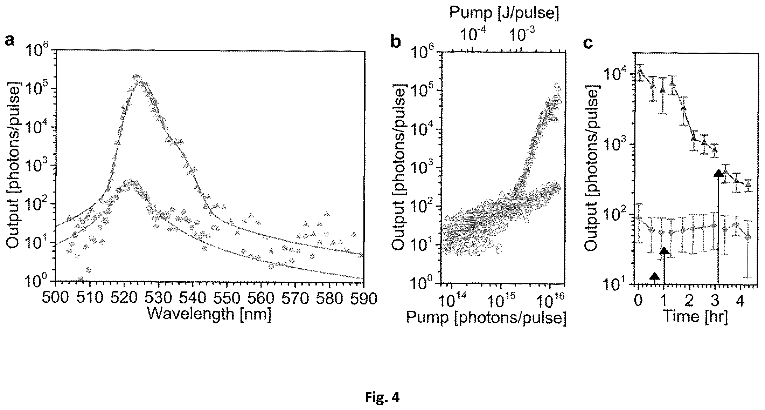

Therefore, an experiment was conducted by adding a monoclonal antibody (cp-mAb, illustrated in Figure 1a) to the reservoir feeding the gain medium, which is a ligand bound specifically by the five gene 3 coat proteins at one end of the phage, and monitoring the effect on the laser system (Figure 4).

For both V2 and F2, threshold and spectral measurements were acquired before (Figure 3) and after (Figure 4a, b; V2 only) the addition of cp-mAb. Between these measurement sets, for V2 only, intensity data were collected at three different levels of pumping 15 min after adding either a buffer control or cp-mAb into the reservoir containing the detection probes (Figure 4c).

For V2, lasing could no longer be sustained with pump pulses of $1.4 \times 10^{16}$ photons/pulse after the addition of 90 fmol/mL cp-mAb to the gain medium, resulting in a 690-fold decrease in the output at 524.8 nm (Figure 4a). The 90 fmol/mL cp-mAb was added to the reservoir feeding the gain medium in three steps, so the intensity data reveal the response of the viral laser to these step increases in cp-mAb. The addition of 9 fmol/mL cp-mAb to the gain medium and then another 20 fmol/mL cp-mAb triggered an increase in the threshold point of V2 as the antibody mixed and bound to the surface of the lasing detection probes (Figure 4c). The threshold point reached a new steady state position after approximately 70 min with no further addition of cp-mAb, but this was then disturbed following the addition of a further 61 fmol/mL cp-mAb, which coincided with the cessation of lasing (Figure 4c).

The initial 5.5-fold step-change at a pump level of $1.3 \times 10^{16}$ photons/pulse was in response to 29 fmol/mL cp-mAb, which equates to 780 lasing detection probes per 1 target biomolecule (Figure 4c), and when the pumping was below the laser threshold the addition of cp-mAb had no effect on the output, indicating that lasing was crucial to the responsivity. The antibodies bound to the phage would red-shift the absorption spectra of certain dyes due to solvent exclusion and contact with a dielectric body with polar side-chains. Theoretical studies of 5 $\mu$M/mL donor dye in bulk solution doped with 0.1 $\mu$M/mL acceptor dye suggest that these red-shifted dyes would sink energy by cavity-enhanced energy transfer and FRET, increasing the threshold point. This effect would be enhanced by homo-FRET transfer between dyes on the same phage, and absorption of the emission by ground state dyes on the antibody-bound phage. This experiment is the first empirical evidence that a biolaser can detect a medically-relevant class of biomolecule at clinically-relevant concentrations below the probe concentration.

Since the fraction of ligands bound by probes initially increases at a rate determined by the probe concentration and the on rate, which is typically ~ $10^4$ M$^{-1}$ s$^{-1}$ for an antibody, mix-and-measure ligand-binding assays with a large excess of probes could rapidly detect sub-1 fmol/mL ligand concentrations, which is often not practical for nanosensors or surface-based methods due to long accumulation times. ELISA, which involves binding the ligands to surface-immobilized antibodies before adding a large excess of a second antibody which binds a different epitope of the surface-bound ligands, can achieve detection limits between 1 pmol/mL and 0.1 amol/mL depending on the antibody-antigen affinity, but unbound probes must be washed away making this technique laborious, disruptive to the biological system and unsuited to continuous monitoring applications.

In the control experiment, F2 was only marginally responsive to the addition of 9.1 pmol/mL cp-mAb, showing that fluorescein does not bind cp-mAb and that the lasing performance is not inherently affected by the target biomolecule or by any other molecules or salts in the solution. Other explanations for the observed changes in the threshold dynamics, including photobleaching, dilution of the probes due to the filter and the addition of buffer, and air bubbles have been ruled out.

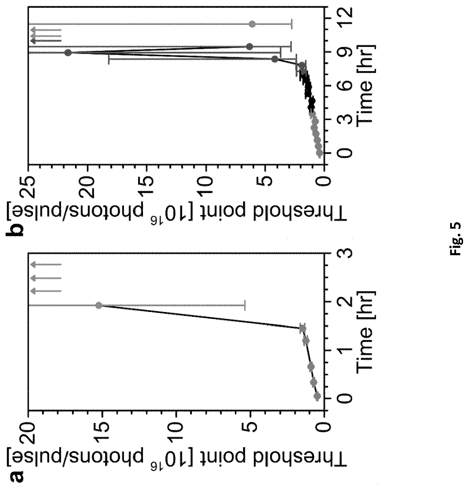

In two further experiments in R2, the threshold behaviour of dye-labelled M13 was monitored as a function of time and the addition of cp-mAb or a non-binding antibody. For each experiment, the data were fit globally using equation (30) with parameters $\chi_0$ and $\chi_1$ shared between each set of threshold measurements. For further experiment 2, 20 pmol/mL V2 contained no antibody (blue), before 4.5 pmol/mL cp-mAb was added (green). For further experiment 1, 23 pmol/mL V2 initially contained no antibody (blue), 91 fmol/mL mouse IgG2a isotype control was added (black) and then cp-mAb was added in two steps so that the concentration of cp-mAb was initially 91 fmol/mL (purple) and then increased to 1.9 pmol/mL (green). See Methods for more details. The threshold points derived from the fitted models have been plotted against time in Figure 5a for further experiment 2 and in Figure 5b for further experiment 1. Measurements have been represented by an arrow if the fitting implied that the sample could no longer sustain lasing. Error bars that extend beyond the upper limit of the y-axis extend to infinity.

In two further experiments, photobleaching caused a steady increase in the threshold point of the viral laser as time elapsed without any step-changes, and in further experiment 1 there was no response to the addition of a non-binding control antibody (mouse IgG2a isotype control) (Figure 5). In both experiments, the subsequent addition of the cp-mAb ligand resulted in a step-change in the threshold point confirming the specificity of the ligand-binding assay. The effect is more pronounced and immediate in further experiment 2 than in further experiment 1, consistent with more cp-mAb being adding in further experiment 2.

In further experiment 1, the addition of a non-binding control antibody did not appear to result in non-specific binding. However, in other assays systems, non-specific binding by the probes to other biomolecules or surfaces could result in modulation of the optical response of the viral laser which would be different to distinguish from modulation due to specific binding by the probes to their target ligand. This problem could be resolved by analysing the modulation of the optical response over time. Since the biomolecule virus compounds formed by the binding of the probes to the target ligand would exist in dynamic equilibrium with unbound virus and unbound target biomolecule, the number of biomolecule virus compounds would vary over time. Since the number of compounds would vary over time, the modulation of the optical response would also vary over time. The autocorrelogram of the optical response over time would depend on the affinity of the virus for its target biomolecule. The affinity of the virus for its target biomolecule would be greater for its target biomolecule than for all other biomolecules in solution, providing a unique signature for distinguishing between high affinity specific binding and low affinity non-specific binding. If the optical excitation was modulated about the threshold point excitation power, then the frequency response of the output would contain harmonics since the response of the viral laser to pumping about the threshold point is non-linear. The amplitude of these harmonics might be very sensitive to the formation of virus biomolecule complexes, which might aid the autocorrelation analysis. The harmonics might also have sidebands due to amplitude modulation of the sample by effects such as photobleaching.

Viral lasers are the only category of biolaser that can be genetically programmed to selectively bind a broad range of target biomolecules and chemically modified to deliver a five decade increase in signal-to-noise, 4 nm emission linewidth and large, non-linear responses in output to small changes in probe concentration. The additional optics required for lasing could be readily integrated alongside contemporary optical technologies such as compact pump lasers and photodetectors into existing fluorimeter platforms, making them into powerful tools for digital biomedicine. Our proof-of-concept experiments indicate that viral lasers can be used to measure clinically-relevant concentrations of biomolecules with a sensitivity approaching that of ELISA, yet without the need for probe immobilisation or multiple wash steps. This paves the way for genetic reprogramming of viral lasers to selectively bind new target biomolecules directly in solution, which would minimise disruption of the biological system under scrutiny.

The Methods section contains more detail on the sample preparation, the free-space optical assembly, a description of the further experiments in resonator R2, the characterisation of the laser and optical properties, the theoretical models and data analysis. There is also an extended technical description of proof-of-concept mix-and-measure ligand-binding assay.

## Methods

**Sample preparation.** The M13 stock was prepared from a single plaque to ensure genetic homogeneity. M13 was amplified in Top10F' *E. coli* cultures and purified by several rounds of centrifugation with or without polyethylene glycol (PEG-8000) and sodium chloride to either selectively precipitate the phage or to remove cells and cellular debris. The M13 solutions were filter-sterilised with a 0.22 $\mu$m filter. For the experiments performed in the resonator R1, 24.9 mg fluorescein isothiocyanate isomer 1 (FITC) powder was added to 50 mL 2.0 mg/mL M13, 100 mM sodium borate, pH 9.1, and incubated for 2 hr at 37 °C prior to quenching with 20 mL 1 M tris-HCl, pH 7.5. For the experiments performed in R2, 15.5 mg FITC powder was added to 29.8 mL 0.64 mg/mL M13, 100 mM sodium borate, pH 9.2, and incubated for 4 hr 15 min at 37 °C prior to quenching with 90 mL 133 mM tris-HCl. In both procedures, the dye was removed by rounds of centrifugation with and without PEG-8000/NaCl. The precipitated M13 was resuspended in 100 mM sodium borate, pH 9.0. For the dye-labelled M13 used in the resonator R2, the solution was also driven through a 0.22 $\mu$m filter.

The M13-binding antibody (Life Technologies, M13 phage coat protein monoclonal antibody from clone E1) which recognises the five g3p coat proteins at one end of M13 and the non-binding antibody (Life Technologies, Mouse IgG2a isotype control) were aliquoted and stored at -20 °C at a concentration of 1 mg/mL. Before use, the antibody stock would be thawed prior to dilution to the appropriate concentration. The antibody was added to the reservoir of the gain medium by pipetting the antibody onto the side-wall of the reservoir and washing the side-wall with the sample ejected from the silicone tubing connected to the flow cuvette. The antibody was allowed to mix with the sample solution as it circulated between the reservoir and the flow cuvette prior to measurement for 15 min for V2 (Figure 4) and for 20 min for F2. The buffer control was added in the same way (Figure 4). The molecular mass of the M13-binding antibody was assumed to be approximately 160 kDa.

**Free-space optical assembly.** The resonant cavities were pumped with pulses of 493 nm light with a FWHM (Full Width at Half Maximum) of 3 ns to 5 ns from an optical parametric oscillator (EKSPLA, NT 342B-20-AW) in single-shot mode via beam-shaping optics consisting of spherical (Comar Optics, 200 PB 25 and 100 NB 25) and cylindrical (Comar Optics, 100 YB 40 and 25 UB 25) BK7 crown-glass lenses with anti-reflection coatings, which transformed the 4.5 mm diameter circular beam cross-section to a 2 mm × 9 mm ellipse. The pulse intensity was controlled with two Glan-Laser calcite polarizers (Thorlabs, GL10). The first was mounted in a motorised rotation stage (Thorlabs, PRM1/MZ8) connected to a T-cube DC servo controller (Thorlabs, TDC001) and the second was fixed with its polarization axis parallel to the polarization of the excitation pulses. The pump energy was monitored by partially reflecting the light with either, in the resonator R1, a pellicle beamsplitter (Thorlabs, BP208), or, in the resonator R2, a glass window, towards a pyroelectric probe (Molectron, J25) connected to a Joulemeter (Molectron, EM500). In R2, a plano-concave, f = -50 mm lens (Newport, KPC040) was placed before the pyroelectric probe to match the widths of the beam and the sensor. In the resonator R1, the detector electronics was triggered by the electronic trigger from the pump source; in the resonator R2 it was triggered by an optical trigger, consisting of a photodiode (Thorlabs, SM1PD1A) connected to a high-speed circuit preceded by an OD 2 neutral density filter (Thorlabs, NDUV20B) with minor damage to the optical coating, positioned behind a protected aluminum mirror (Thorlabs, PF10-03-G01) in the path between the beamsplitting window and the pyroelectric probe. Additionally, for the resonator R2, the pump was transmitted through a rectangular aperture that matched the dimensions of the cuvette window, and a dielectric shortpass filter with a cut-off wavelength of 510 nm (Comar Optics, 510 IK 50).

The pump impinged on a 2 mm × 8 mm window to a flow cuvette (Starna, 583.2.2FQ-10/Z15) with a 2 mm × 2 mm × 10 mm internal chamber, so the maximum path length of the pump light through the cuvette was 2 mm since the long axis of the internal chamber was orthogonal to the direction of the pump beam and parallel to the axis of the resonant cavity. The samples or cleaning fluids were flowed into the cuvette via silicone tubing from reservoirs using a peristaltic pump (RS Components), and samples would then flow back into the reservoir. The flow rates were 2.5 mL/min for resonator R1 and 3.0 mL/min for R2, and the time between pulses was long enough for the dye in the cuvette to be refreshed. For resonator R2, there was a 5 μm in-line filter (Whatman, Polydisc HD 5.0 pm) between the reservoir and the cuvette. In further experiment 2 in resonator R2, the in-line filter was removed and the flow rate was increased to 4 mL/min. The cuvette had 2 mm × 2 mm Spectrosil quartz windows on opposing walls at 90° to the entrance window, which were aligned with the axis of the resonant cavity. The Fresnel reflectance at the glass interface would have been,

$$(3) \, r = \left(\frac{n_1 - n_2}{n_1 + n_2}\right)^2$$

so that **r = 0.0350** at the glass-air interface for **n(fused-silica) = 1.46** and **n(air) = 1.00,** and **r = 0.00217** at the glass-water interface for **n(water) = 1.33.** Consequently, the transmission through the cuvette was nominally 0.860 per round trip of the resonant cavity.

The resonator R1 comprises a flat dielectric output coupler (Comar Optics, 25 MX 02) and a dielectric spherical mirror with a radius of curvature of 400 mm (Thorlabs, CM508-200-E02), separated by 300 mm with the cuvette adjacent to the output coupler. In the resonator R2, the spherical mirror was replaced with a compound optic consisting of a protected silver spherical mirror with a radius of curvature of 50 mm (Thorlabs, CM254-025-P01) bonded to a 300 μm pinhole (Thorlabs, P300S) and the separation of the mirrors was reduced to 26 mm. The reflectivity of the flat mirror was 99 % and the reflectivities of the dielectric and metallic spherical mirrors were 99.6 % at 530 nm unpolarized and 98.8 % at 520 nm unpolarized, respectively, based on product data from the manufacturers. The fraction of the oscillation retained is then nominally 0.848 per round trip for resonator R1, and 0.841 per round trip for resonator R2. The round-trip time for light oscillating between the mirrors of resonant cavities was 2.0 ns for resonator R1 and 195 ps for resonator R2, for a cuvette filled with water. The diameters of the mirrors were much greater than the diameters of the cuvette windows and the pinhole, so the Fresnel number, **F**

$$(4) \, F = \frac{a^2}{L \, \lambda}$$

where **a** is the radius of the aperture and **L** is the length of the resonant cavity, was approximately

$$\frac{(1\text{ mm})^2}{300\text{ mm}\times 530\text{ nm}} = 6.3$$ for resonator R1 and $$\frac{(0.3\text{ mm})^2}{26\text{ mm}\times 520\text{ nm}} = 6.7$$ for resonator R2. In both cases, **F > 1,** so large diffraction losses are not anticipated.

The effective volume of the gain medium is the volume that is pumped which can contribute to the laser emission. For resonator R1, this is the entire volume of the cuvette chamber that is subjected to pumping, $V_{eff}$ **= 8 mm $\times$ 2 mm $\times$ 2 mm = 32 $\mu$L.** For resonator R2, this volume is reduced due to the pinhole before the spherical mirror. The resonator R2 has a half-confocal resonator geometry, so the radius of the beam at the flat mirror is expected to be a factor of $\sim \frac{1}{\sqrt{2}}$ of the radius at the spherical mirror, which is **r $\sim$ 150 $\mu$m** due to the pinhole.

Using similar cones, the effective volume of the gain medium $V_{eff}$ that is pumped is given by,

$$(5)\, V_{eff} = \frac{\pi}{6}\left(\frac{r}{L}\right)^2 \left(\sqrt{2}-1\right)^2 \left\{\left(\frac{L}{\sqrt{2}-1}+l_2\right)^3 - \left(\frac{L}{\sqrt{2}-1}+l_1\right)^3\right\}$$

where $l_1$ is the separation of the flat mirror and the closest edge of the pumped volume, and $l_2$ is the separation of the flat mirror and the furthest edge of the pumped volume. For $l_1 \sim$ **4.5 mm,** $l_2 \sim$ **12.5 mm** and **L $\sim$ 26 mm,** $V_{eff}$**(R2) $\sim$ 0.365 $\mu$L.**

The absorption of the pump by the gain medium depends on the optical path length of the gain medium in the direction of the pump. For resonator R2, the mean optical path length $l_{mean}$ can be estimated by calculating the mean path length across a cylinder with the same length and volume as the effective volume of the gain medium, so

$$(6)\, l_{mean} = \frac{4}{\pi}\sqrt{\frac{V_{eff}}{\pi\,(l_2-l_1)}}$$

For resonator R2, $l_{mean} \sim$ **153 $\mu$m.** For resonator R1, $l_{mean}$ **= 2 mm,** which is the depth of the cuvette chamber.

The light transmitted through the output coupler was collected with either an unoccluded lens (Newport, KPX112) or a lens coupled with an iris diaphragm (Thorlabs, LA1484-A, and Thorlabs, SM1D12CZ) such that the f/numbers were $$\frac{300\text{ mm}}{22.9\text{ mm}} = 13.1$$ for resonator R1 and $$\frac{300\text{ mm}}{5.5\text{ mm}} = 54.5$$ for resonator R2, respectively. A custom-built spectrometer combining a photomultiplier tube (PMT) (Hamamatsu Photonics, R1166) connected to a high voltage supply (Hamamatsu Photonics, C9525) and a Czerny-Turner monochromator (Bentham, M300EA) with a focal length of 300 mm, f/number of 4.2 and a 1200 grooves/mm grating, recorded the output. Before the spectrometer, the output could be optically attenuated using UV fused-silica reflective neutral density filters with ODs ranging from 0.5 to 3.0 for resonator R1 and 0.5 to 4.0 for resonator R2 (Thorlabs) mounted in a filter wheel working in conjunction with a OD 4.0 filter in a flip mount. Optical feedback was prevented by aligning the flip mount for resonator R1 and resonator R2 and the filter wheel for resonator R2 so that the normal to each filter was not parallel to the direction of the incident light. For resonator R1 the mounts were manual but for resonator R2 they were replaced with motorised equivalents (Thorlabs, FW102C, and Thorlabs, MFF102/M). For resonator R2, optomechanics prevented stray light from entering the spectrometer, and a dielectric longpass filter with a 500 nm cut-on (Thorlabs, FEL0500) connected to the light-collecting lens blocked any scattered pump light. For resonator R2, the bias voltage to the PMT was -860 V because this enabled single photon measurements without compromising linearity.

The voltage across a 50 Q terminator (for resonator R2, Pico Technology, TA051) connected to the PMT was recorded using an oscilloscope (for resonator R1, Tektronix, TDS210 and for resonator R2, Tektronix, TDS3052). The waveforms were transferred to a PC for real-time analysis in a custom program that controlled the optical assembly (National Instruments, LabVIEW 8).

The term "pump" refers to the radiant energy input into the resonant cavities in units of photons/pulse, which can be converted to a spectral irradiance of the resonant cavities via multiplication by $\hbar\omega$/16 mm$^2$/$\delta\lambda$(0.12 nm)/4 ns pulse, since the area of the cuvette window was 16 mm$^2$, the spectral linewidth of the OPO was 0.12 nm and the optical pulse length was 3 ns to 5 ns, and $\hbar\omega$ is the energy of one photon with a wavelength of 493 nm. The term "output" refers to the radiant energy emitted from the resonant cavities in the direction of the detection optics. The reported output is different to the signal observed by the detector by a factor that accounts for the optical attenuation of the spectrometer, and does not account for the radiation that emits in directions that bypass the spectrometer. The oscillation build-up in the resonant cavity would have been greater than the measured output. The output from resonator R2 was calibrated by measuring the response of the detection electronics to single photons, so it is reported in units of photons/pulse.

Equivalently, the output from resonator R2 can be converted to a spectral radiance emitted from the resonant cavity via multiplication by $\hbar\omega/1.3 \times 10^{-3}$ sr/$\delta\lambda$(0.4 nm)/pulse, where the solid angle of the spectrometer is $1.3 \times 10^{-3}$ sr and the linewidth of the monochromator is approximately 0.4 nm, and $\hbar\omega$ is the energy of one photon at the selected wavelength of the monochromator.For resonator R2, optomechanics and a filter prevented light from external sources to the resonant cavity from entering the spectrometer, but this was not implemented for resonator R1. Consequently, resonator R1 could not be calibrated by measuring the response of the detection electronics to single photons. Instead, the output has been reported in arbitrary units proportional to the number of photons emitted from the resonant cavity in the direction of the detection optics.

**Description of further experiments in resonator R2.** Two additional experiments were performed in resonator R2 using the same dye-labelled M13 sample to test whether photobleaching could cause the step-changes in the output observed in Figure 4. Further experiment 1 was similar to the experiment described previously, except that 91 fmol/mL mouse IgG2a isotype control, which should not be bound by M13, was added to the gain medium before 91 fmol/mL cp-mAb was added, which was then increased to 1.9 pmol/mL. Buffer was not added to the reservoir between measurements and the antibody was allowed to mix for at least 12 min prior to measurement. Further experiment 2 was similar to the previous experiments, except that the dye-labelled M13 was diluted by a factor of ~ 0.89 in type 1 water and the final sample volume was ~ 2.75 mL, which was less than the 6.85 mL used in previous experiments, and the in-line filter was removed. Also, prolonged storage of the dye-labelled M13 at 4 °C resulted in some sedimentation, so the solution was transferred to a fresh container and then centrifuged to remove any residual insoluble material. For this experiment, only 4.5 pmol/mL cp-mAb was added. The changes in the threshold dynamics with time before and after the addition of antibody were measured in both experiments. Emission spectra before and after the addition of antibody were acquired for further experiment 2 but not for further experiment 1.

**Characterisation of laser properties.** The threshold dynamics were recorded by measuring the output at a fixed wavelength at variable pump levels. The intensity of the pump pulses were controlled by rotating the first polarizer. If the output was outside of the linear range of the spectrometer, the optical attenuation was adjusted to compensate and the measurement was repeated. In resonator R1, the wavelength was fixed to 527.0 nm and 528.0 nm for the measurements of fluorescein and dye-labelled M13, respectively. In resonator R2, the wavelength was fixed to 520.5 nm and 523.5 nm for the measurements of fluorescein and dye-labelled M13, respectively, except for further experiment 2, for which the wavelength was fixed to 525.0 nm. The spectral measurements were acquired by fixing the pump level and recording the output at different emission wavelengths by rotating the monochromator grating.

**Characterisation of optical properties.** The absorption spectra were recorded using a UV-Vis spectrophotometer (for experiments using resonator R1, Hitachi, Digilab U-1800; for experiments using resonator R2, Thermo Scientific, Nanodrop 2000C) with 1 cm path length cuvettes. The mass of a single M13 is taken to be 16.4 MDa $\pm$ 0.6 MDa (error is 2 standard deviations from the mean) which is the reported mass of fd virus based on measurements of the translational diffusion coefficient, sedimentation coefficient and density increment. The extinction coefficient of M13 is taken to be 3.84 $\pm$ 0.06 mg$^{-1}$ cm$^2$ at 269 nm (error is 95 % confidence limit) which is the reported extinction coefficient of fd virus in KCl/P buffer. Based on these values, the extinction coefficient used to determine the concentration of M13 was $6.30 \times 10^7$ M$^{-1}$ cm$^{-1}$ at 269 nm. The absorption of both the M13 and the fluorescein at both the dye peak and at 269 nm were accounted for in the calculations of the concentration of dye-labelled M13 and the number of attached dyes. The precision of the stated concentrations was limited by the precision of the pipettes used (Gilson, Pipetman) and the precision of the UV-Vis spectrophotometers.

For samples measured in experiments in resonator R2, the extinction coefficient of 34.0 $\mu$g/mL fluorescein, 100 mM sodium borate, pH 9.0 was calculated to be 82752 M$^{-1}$ cm$^{-1}$ at the peak absorption at 490 nm and 977 M$^{-1}$ cm$^{-1}$ at 269 nm; the extinction coefficient of M13 at the absorption peak for the attached dyes at 494 nm was $1.3 \times 10^6$ M$^{-1}$ cm$^{-1}$. The extinction coefficients of fluorescein attached to M13 were assumed to be the same as for unattached fluorescein at 269 nm and at the absorption peak. The concentration of the dyes attached to the dye-labelled M13 was 12.8 nmol/mL and the concentration of dye-labelled M13 was 23 pmol/mL, so there were 564 dyes per M13 on average. The fluorescein was diluted by a factor of 0.13 before further measurements were acquired, so its final concentration was 13.3 nmol/mL.

For samples measured in experiments in resonator R1, the extinction coefficient of 100 ug/mL fluorescein, 100 mM sodium borate, pH 9.0 was calculated to be 82413 M$^{-1}$ cm$^{-1}$ at the peak absorption at 491 nm and 10634 M$^{-1}$ cm$^{-1}$ at 269 nm; the extinction coefficient of M13 at the absorption peak for the attached dyes at 493 nm was determined to be $1.4 \times 10^6$ M$^{-1}$ cm$^{-1}$. The concentration of the dyes attached to the dye-labelled M13 was calculated to be 159 nmol/mL and the initial concentration of dye-labelled M13 was 413 pmol/mL, so there were 386 dyes per M13 on average. The initial concentration of fluorescein was 301 nmol/mL. For both resonator R1 and resonator R2, the wavelength of the pump was 493 nm. For resonator R1, the extinction coefficients at 493 nm compared to the extinction coefficient at the absorption peaks were a factor of 0.976 and 1 for fluorescein and dye-labelled M13, respectively. For resonator R2 these factors were 0.984 and 0.991, respectively. The separation of fluorescein dyes in solution was calculated by taking the cube root of the mean volume per dye. **Theoretical models.** The changes in the energy build-up in a resonator mode and in the upper state population of the dyes can be modelled using the two rate equations:

$$(7) \frac{dn}{dt} = K_{RAD} \, D_2 \, (n + n_0) + K_{RAD} \, D_2 - K_L \, n$$

$$(8) \frac{dD_2}{dt} = K_F \, \varphi \, (D - D_2) - K_{RAD} \, D_2 \, (n + n_0) \, \xi - K_2 \, D_2$$

where $n$ is the energy in the resonator mode, $\varphi$ is the rate of pumping of the resonator mode, $D$ is the number of dyes in the resonator mode volume, $D_1$ and $D_2$ are the number of dyes in the lower and upper state of the laser transition, respectively, $K_F$ is the strength of the coupling of the pump with the dyes, $K_{RAD}$ is the rate of emission into the mode per dye, $K_2$ is the rate of decay from the upper state per dye, $K_L$ is the rate of loss from the resonator, $n_0$ is the seed fluorescence and $\xi$ is the asymmetry factor which is typically equal to 1.

In eq. (7), the first and second term describe the rate of stimulated and spontaneous emission, and the third term describes the rate of loss from the resonator. In eq. (8), the first term describes the rate of pumping of the dyes, and the second and third terms describe the depopulation of the upper state due to stimulated emission and all other sources, respectively.

The rate of pumping is derived from the Beer-Lambert law assuming that every pump photon absorbed results in a dye in the upper state:

$$(9) \frac{dD_2}{dt} = \varphi \left( 1 - e^{-\frac{D_1}{v} \varepsilon \, l} \right) \sim \varphi \, D_1 \frac{\varepsilon \, l}{v} = K_F \, \varphi \, (D - D_2)$$

where $\varepsilon$ is the extinction coefficient, $l$ is the optical depth of the resonator mode and $v$ is the volume of the resonator mode that is being pumped. This approximation is valid for resonator R2 because the concentration of the dyes is low and the path length across the resonant cavity mode is narrow. For resonator R1, these assumptions do not strictly hold, but are necessary to simplify the equations so that they can be solved analytically. Additionally, the Beer-Lambert law assumes that the dyes are homogeneously distributed in the solution, which is not the case for gain media composed of dye-labelled M13.

A full description of the threshold dynamics would require a rate equation for the energy build-up in each resonator mode, but consideration of just a single mode is sufficient to model the key aspects of the system. Consistent with this approach, the volume of the resonator mode being pumped $v$ is set equal to the effective volume of the gain medium being pumped $V_{eff}$.

The rate of radiative emission per dye into the resonator mode is a function of the rate of spontaneous emission and the number of resonator modes:

$$(10) \qquad K_{RAD} = \frac{\gamma_{RAD}}{\rho}$$

where $\gamma_{RAD}$ is the rate of spontaneous emission per dye and $\rho$ is the number of resonator modes.

The rate of decay from the upper state per dye is the sum of the rate of spontaneous emission and the rate of non-radiative decay:

$$(11) \quad K_2 = \gamma_{RAD} + \gamma_{NON}$$

where $\gamma_{NON}$ is the rate of non-radiative decay per dye.

$K_L$ is determined by the loss rate of the resonator, which is given by

$$(12) \quad K_L = -\frac{1}{\tau_r} \ln(1 - LOSS)$$

where $\tau_r$ is the round-trip time and **LOSS** is the fraction of the oscillation build-up lost per round-trip.

The energy in the resonator mode is derived from eq. (7) and eq. (8) by assuming steady state emission, eliminating $D_2$ and solving the resulting quadratic equation:

$$(13) \quad n = \frac{\beta}{2\,\xi\,K_{RAD}} \left\{ K_F\,\varphi \left( \frac{D\,K_{RAD}}{K_L} - 1 \right) - \xi\,K_{RAD}\,n_0 - K_2 + \right.$$
$$\left. \sqrt{\left( K_F\,\varphi \left( \frac{D\,K_{RAD}}{K_L} - 1 \right) - \xi\,K_{RAD}\,n_0 - K_2 \right)^2 + \frac{4\,K_F\,\varphi\,D\,\xi\,K_{RAD}^2}{K_L}\,(n_0 + 1)} \right\}$$

where $\beta$ is the optical attenuation due to the output coupler and the spectral bandwidth of the spectrometer.

The build-up of energy in the dominant resonator mode depends on optical amplification, which implies the presence of a non-zero seed fluorescence $n_0$ prior to amplification. This is modelled as the energy in the resonator mode when there is no stimulated emission. From eq. (7) and eq. (8):

$$(14) \quad \frac{dn_0}{dt} = \theta\,\gamma_{RAD}\,D_2 - K_L\,n_0$$

$$(15) \quad \frac{dD_2}{dt} = K_F\,\varphi\,(D - D_2) - K_2\,D_2$$

where $\theta$ is the fraction of the spontaneous emission that emits at a wavelength and in a direction such that it can contribute to the seed fluorescence. Using the same assumptions as for eq. (13):

$$(16) \quad n_0 = \frac{D\,\theta\,\gamma_{RAD}}{K_L \left( 1 + \frac{K_2}{K_F\,\varphi} \right)}$$

There is a background fluorescence from dyes that are pumped but are not contained within the resonator mode volume. As for the seed fluorescence, there is no stimulated emission, so eq. (7) and eq. (8) are reduced to:

$$(17) \quad \frac{dn}{dt} = \mu\,\gamma_{RAD}\,D_2' - K_L'\,n$$

$$(18) \quad \frac{dD_2'}{dt} = K_F'\,\varphi'\,(D' - D_2') - K_2'\,D_2'$$

where the apostrophe indicates that the variable or parameter is different for the dyes outside of the resonator mode volume compared to the dyes inside of this volume. For instance, for resonator R2 the volume of the cuvette chamber that overlaps the resonator mode volume is a small fraction of the total cuvette chamber volume, so **D' ≫ D.** Likewise, the fraction of the pump $\varphi$ that impinges on the mode volume is small. The change in volume also accounts for the change in **$K_F$,** see eq. (9).

There is no optical feedback so the background fluorescence is lost from the resonator at the rate $K_L'$ which light can travel out of the resonator. In this model, **$K_2$** is allowed to differ from $K_2'$ because the rate of non-radiative decay may be different for dyes inside and outside of the resonator mode since there are more channels for interaction between dyes inside the resonator mode. **$\mu$** is the fraction of the spontaneous emission from dyes outside of the resonator mode volume that enters the spectrometer, which is dependent on the solid angle subtended by the light-collecting lens.

Using the same assumptions as for eq. (13) and eq. (16), the background fluorescence is given by

$$(19) \quad n = \frac{D'\,\mu\,\gamma_{RAD}}{K_L' \left( 1 + \frac{K_2'}{K_F'\,\varphi'} \right)}$$

Light may have entered the spectrometer from external sources to the resonant cavity, which is accounted for by a constant offset.

The threshold point $\varphi_{th}$ is given by

$$(20) \quad K_F \, \varphi_{th} \left( \frac{D \, K_{RAD}}{K_L} - 1 \right) - \xi \, K_{RAD} \, n_0 - K_2 = 0$$

from eq. (13). Including the term for the seed fluorescence, this is then

$$(21) \quad K_F \, \varphi_{th} \left( \frac{D \, K_{RAD}}{K_L} - 1 \right) - \xi \, K_{RAD} \, \frac{D \, \theta \, \gamma_{RAD}}{K_L \left( 1 + \frac{K_2}{K_F \, \varphi_{th}} \right)} - K_2 = 0$$

which can be solved to find

$$(22) \quad \varphi_{th} = \frac{K_2 \, (2 \, K_L - D \, K_{RAD}) + D \, K_{RAD} \left\{ \theta \, \gamma_{RAD} \, \xi + \sqrt{(\theta \, \gamma_{RAD} \, \xi - K_2)^2 + \frac{4 \, \theta \, \gamma_{RAD} \, \xi \, K_2 \, K_L}{D \, K_{RAD}}} \right\}}{2 \, K_F \, (D \, K_{RAD} - K_L)}$$

From eq. (11), this can be simplified by noting that

$$(23) \quad \theta \, \gamma_{RAD} \, \xi \, \ll \, K_2 = \gamma_{RAD} + \gamma_{NON}$$

since

$$(24) \quad \theta \, \xi \, \ll \, 1$$

and that for gain to exceed losses

$$(25) \quad \frac{D \, K_{RAD}}{K_L} > 1$$

so that the threshold point is given by

$$(1) \quad \varphi_{th} = \frac{K_2 \, K_L}{K_F \, (D \, K_{RAD} - K_L)}$$

The minimum number of dyes required to achieve threshold $D_{min}$ even at very large levels of pumping, is given by

$$(26) \quad D_{MIN} = \frac{K_L}{K_{RAD}}$$

From eq. (13), the output far above threshold when $\varphi \to \infty$ is given by

$$(27) \quad \frac{n}{\varphi} \to \frac{\beta \, K_F}{\xi \, K_{RAD}} \left( \frac{D \, K_{RAD}}{K_L} - 1 \right)$$

If there is no gain depletion above threshold, then $\xi = 1$,

$$(2) \qquad \frac{n}{\varphi} \rightarrow \frac{\beta\,K_F}{K_{RAD}}\left(\frac{D\,K_{RAD}}{K_L} - 1\right)$$

Furthermore, if $D \gg D_{min}$ then

$$(28) \qquad \frac{n}{\varphi} \rightarrow \frac{\beta\,K_F\,D}{K_L}$$

and the gain above threshold ceases to depend on the rate of spontaneous emission.

The time-dependence of the pump energy was accounted for by assuming a Gaussian profile with a FWHM of 4 ns,

$$(29) \qquad \varphi(t) = \varphi_0 \times Gaussian(t)$$

and calculating the output based on the peak pump energy. The non-linear response of output to increased pumping means that the peak pump energy has the greatest contribution to the overall output. An alternative method was tested in which the pump pulse was split into sub-nanosecond time bins over a ~ 50 ns time window centred about the pulse peak and the output calculated by summing the outputs from each time bin, but the results generated were negligibly different to the preferred method. The calculation of the threshold point accounted for the time-dependence of the pump energy.

For resonator R2, only $\frac{1}{2\,mm}$ of the pump impinged on the resonator mode because its diameter was less than the 2 mm height of the window. The remainder would have pumped dyes outside of the dominant resonator mode.

The threshold point for each set of data for a different concentration of dye-labelled M13 or fluorescein could be readily determined using a model similar to eq. (13),

$$(30) \qquad n = \chi_0 + \chi_1\left\{\left(\frac{\varphi}{\varphi_{th}} - 1\right) + \sqrt{\left(\frac{\varphi}{\varphi_{th}} - 1\right)^2 + \chi_2\,\varphi}\right\}$$

where $\chi_n$ are fit parameters that are not directly linked to the experimentally observable variables, $\varphi_{th}$ is the threshold point and the time-dependence of the pump is disregarded.

**Data analysis.** For resonator R1, the threshold dynamics of fluorescein and dye-labelled M13 were analysed by globally fitting each set of data with a model that employed eq. (13) as well as eq. (16) for the seed fluorescence. The parameters $\gamma_{RAD}$, $K_L$, $K_F$, $I_{mean}$, $V_{eff}$ and $\xi$ were all fixed and the same for each set of measurements. Each of these were calculated in advance (see Free-space optical assembly and Theoretical Models), except for $\gamma_{RAD}$ which is known to equal 0.240 ns$^{-1}$.

**D** was fixed but different for each set of measurements. A factor was calculated from the spectral measurements to account for the difference between the output at the measured emission wavelength and the spectral peak, which was fixed for each set of measurements except for 146 nmol/mL fluorescein, since there was no spectral measurement of this sample and it may have undergone a spectral shift. The parameters $\rho$, $\beta$, $\theta$ and a constant offset to account for external light sources were not fixed and were shared between each set of data because they should be consistent between measurements. The parameter $\gamma_{NON}$ was not fixed and shared between the sets of dye-labelled M13 measurements, but allowed to vary individually for each set of fluorescein measurements.

In Figure 2c, the line representing the threshold point as a function of probe concentration was simulated using this model. In Figure 2d, the pump energy values were selected because they represent local minima in a plot of pulse energy variability against pulse energy. The pulse energy varies from pulse to pulse due to variability in the intensity of the emission from the OPO. The lines were simulated using the same model.

The threshold dynamics of fluorescein and dye-labelled M13 in resonator R2 were analysed using a similar model to resonator R1. Since the effective mode volume was smaller, eq. (19) was employed to account for the background fluorescence from dyes outside of the mode volume. The additional parameters $K'_L$ and $K'_F$ were fixed and the same for each set of measurements, and **D'** was fixed but different for each set of measurements. $\gamma'_{NON}$ was fixed at zero for each set of measurements and the additional parameter $\mu$ was not fixed but shared between each set of measurements. The parameter $\gamma_{NON}$ was not fixed and allowed to vary individually for each set of measurements, except for the fluorescein measurement that did not contain cp-mAb for which

it was fixed to zero. The parameter $\xi$ was not fixed and shared between the dye-labelled M13 measurements, but fixed to 1 for the fluorescein measurements. The reported error on $\xi$ is the standard error.

For experiments conducted in resonator R1, the threshold measurements were additionally fit using eq. (30) to accurately determine the position of the threshold point. From eq. (1), as $D \rightarrow D_{MIN}$, $\varphi_{th} \rightarrow \infty$ and if $D < D_{MIN}$, then $\varphi_{th} < 0$, so the parameter $\varphi_{th}$ was substituted for its reciprocal in the fitting function and subsequently derived after the model converged. The error bars are standard errors. The error bar in Figure 2c that extends beyond the upper limit of the y-axis extends to infinity, and $\varphi_{th} < 0$ for the most dilute dye-labelled M13 sample, implying that the probe concentration was insufficient to achieve lasing.

For the further experiments in resonator R2, the threshold measurements were fit using eq. (30), but parameters $\chi_1$ and $\chi_2$ were shared between sets of data from the same experiment. From comparison of the parameters in eq. (30) and eq. (13) with zero seed fluorescence and with $\xi = 1$, we expected $\chi_1$ and $\chi_2$ to be consistent between these measurements.

For experiments conducted in resonator R1, when the pumping was above the threshold for lasing the spectra were fit to a model consisting of a sum of two Gaussian functions with a constant offset. For experiments conducted in resonator R2, the above-threshold spectra were fit to a model consisting of a single Voigt function or a sum of two Voigt functions with no constant offset, and the below-threshold spectra were fit to a Lorentzian function with no constant offset. In Figure 3, the scatter points in the spectra are the means of four measurements at each wavelength. The mean and coefficient of variation of the pump energy has been reported for each emission spectrum. The spectral peak centres and linewidths were determined by the model fitting and the reported errors are standard errors.

**Extended technical description of proof-of-concept mix-and-measure ligand-binding assay**

Other experimental factors might have resulted in a decrease in the viral laser output, and we consider three possibilities here. The first and most trivial is the dilution of the dye with the addition of antibody solution. This is ruled out on arithmetic grounds: the 5 $\mu$m pore size of the filter was much larger than M13 and the addition of 100 $\mu$L buffer to the 6.85 mL reservoir would not have had a profound effect unless the initial concentration of probes was less than 1.5 % above the minimum required to achieve lasing. The second is air bubbles in the cuvette chamber. Whilst air did sometimes pass through the flow cuvette between measurements, the system was designed to allow air to escape and the variation of the output did not increase as the mean decreased, which would indicate a trapped bubble partially occluding the resonant cavity. Third, because of the repetitive nature of the experiment, photobleaching effects could produce reductions in output which might be confused with those due to the binding of the antibody. For instance, photobleaching of the dyes most likely caused the decrease in the output below threshold, and the output above threshold between the spectral measurement, the threshold measurement and before adding cp-mAb in the titration measurement. However, photobleaching does not account for the step changes in the output during the titration measurement: there was no decrease below threshold and there were only 295 excitation pulses in total compared to 1340 excitation pulses in total for the preceding spectral and threshold measurements. In further experiment 2, photobleaching did not cause step changes in the threshold point and the addition of cp-mAb led to a sharp increase in the threshold point. In further experiment 1, neither photobleaching nor the addition of a non-binding antibody resulted in step changes in the threshold point, and the subsequent addition of cp-mAb resulted in a sharp increase in the threshold point after a short lag period. The effect of the addition of cp-mAb may have been more immediate in further experiment 2 than in further experiment 1 because more cp-mAb was added. The greater output from the viral laser in further experiment 2 immediately prior to the addition of cp-mAb may also have been a contributory factor.

**Optical configuration diagrams**

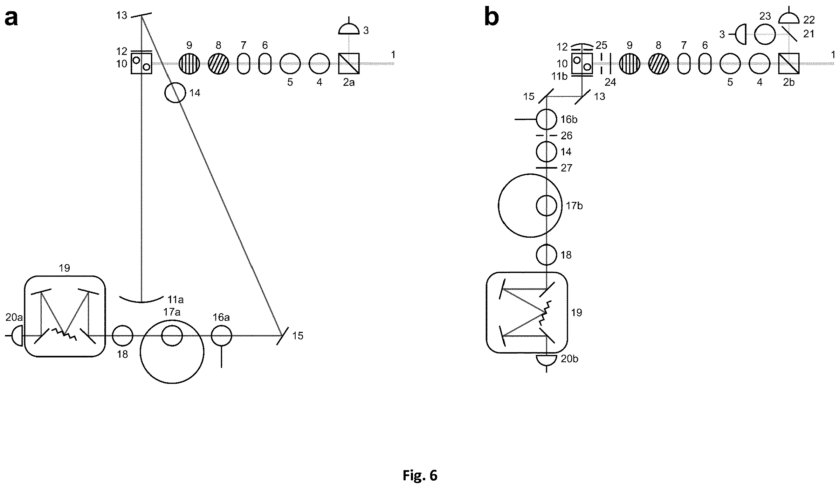

Figure 6 shows the optical configuration for experiments conducted using **a,** resonant cavity R1 and **b,** resonant cavity R2. The numerical labels in **a** and **b** refer to the following list of components:

    1 - Optical parametric oscillator via periscope with two flat 1" Ag mirrors
    2a - Pellicle beamsplitter
    2b - Beamsplitting window
    3 - Beam monitor: pyroelectric probe connected to a Joulemeter
    4, 5 - Spherical beam-expanding telescope
    6, 7 - Cylindrical beam-reducing telescope

8, 9 - Pump energy control: two Glan-Laser calcite polarizers, 8 in a motorised rotation stage, 9 fixed in a manual rotation stage

10 - Flow cuvette with 40 $\mu$l chamber

11a - Spherical dielectric mirror, R = 400 mm

11b - 300 $\mu$m pinhole bonded to a spherical Ag mirror, R = 50 mm

12 - Flat, dielectric output coupler

13 - Flat 1" Ag mirror

14 - 1" spherical convex lens

15 - Flat 1" Ag mirror

16a - OD 4 reflective ND filter in manual flipper mount

16b - OD 4 reflective ND filter in motorised flipper mount

17a - Manual filter wheel mounted with OD 0.5, 1.0, 2.0, 3.0 reflective ND filters, and one unmounted position

17b - Motorised filter wheel mounted with OD 0.5, 1.0, 2.0, 3.0, 4.0 reflective ND filters, and one unmounted position

18 - Spherical convex lens

19 - Monochromator with a 1200 grooves/mm grating

20a - Detector: PMT connected to a 50 Q terminator, voltage measured by a 60 MHz oscilloscope

20b - Same as 20a, except with a 500 MHz oscilloscope and a 1 GHz feed-through 50 Q terminator

21 - Beamsplitting flat 1" Al mirror

22 - Optical trigger to oscilloscope: Si photodiode connected to high-speed circuit preceded by an OD 2.0 reflective ND filter

23 - 1" spherical concave lens

24 - Shortpass filter, $\lambda$ = 510 nm.

25 - Rectangluar aperture

26 - Iris diaphragm

27 - Longpass filter, X = 500 nm

## Claims

1. A method for biosensing a binding ability among a biomolecule, such as an antibody, a membrane protein or the like, and a virus, comprising the steps of:

   a) labelling the virus by conjugating a plurality of dyes, preferably fluorescein dyes, to the virus in order to achieve a labelled virus being controllable with respect to the density of labelled dyes per virus molecule;

   b) solutions-phase-wise binding the labelled virus to the biomolecule in a liquid solution to the extent the binding characteristics among the biomolecule and the labelled virus in order to generate biomolecule virus compounds and to allow for a mix-and-measure ligand-binding homogenous assay that non-disruptively measures the lasing response of the biomolecule virus compounds;

   c) pumping the liquid solution comprising the biomolecule virus compound with light having a wavelength and/or an intensity being adopted to the type of the effective mechanism of the dyes, preferably the effective fluorescence mechanism of the fluorescein dyes, labelled to the virus;

   d) amplifying the light emitted from the pumped dyes in an optical resonator in the attempt to achieve a lasing response from the excited dyes in the optical resonator; and

   e) measuring the intensity of the amplified emitted light and evaluating the intensity of the amplified emitted light against the intensity of the amplified emitted light from a known density of labelled fluorescein dyes per virus molecule or against an equivalent concentration of unbound labelled virus with the same number of dyes per virus in order to determine the binding ability of the biomolecule to the labelled virus.

2. The method according to claim 1 wherein the virus is a M13 bacteriophage or a recombinant Tobacco mosaic virus-like particle (rTMV).

3. The method according to any of the preceding claims, wherein the biomolecule is an antibody or a monoclonal antibody, preferably an IgG2a monoclonal antibody (mAB).

4. The method according to any of the preceding claims wherein the fluorescein dye is a fluorescein isothiocyanate isomer 1 dye.

5. The method according to any of the preceding claims wherein the virus is covalently modified with the fluorescein dye.

6. The method according to any of the preceding claims wherein the pumping is achieved using 1 ns to 20 ns excitation pulses at a wavelength that is within the visible spectrum, preferably in the range of 450 to 600 nm.

7. The method according to any of the preceding claims wherein the liquid solution is circulated between a reservoir and a flow cuvette.

8. The method according to any of the preceding claims wherein the virus is effectively genetically-programmable with a spectral peak emission that is tunable by varying the number of fluorescein dyes attached per virus and/or by modifying the chemical landscape of the surface of the virus.

9. The method according to claim 8, wherein the virus is conjugated with a number of fluorescein dyes equivalent to a range of 0.5 to 2 dyes, preferably 0.7 to 1.2 dyes, on average per ring of coat proteins of the virus.

**Patentansprüche**

1. Verfahren zum Biosensoring einer Bindungsfähigkeit zwischen einem Biomolekül, wie etwa einem Antikörper, einem Membranprotein oder dergleichen, und einem Virus, umfassend die Schritte:

   a) Markieren des Virus durch Konjugieren einer Vielzahl von Farbstoffen, vorzugsweise Fluorescein-Farbstoffen, an das Virus, um ein markiertes Virus zu erhalten, das bezüglich der Dichte markierter Farbstoffe pro Virusmolekül steuerbar ist;
   b) lösungsphasenweises Binden des markierten Virus an das Biomolekül in einer flüssigen Lösung im Umfang der Bindungscharakteristika zwischen dem Biomolekül und dem markierten Virus, um Biomolekül-Virus-Verbindungen zu erzeugen und einen homogenen Mix-and-Measure-Ligandenbindungs-Assay zu ermöglichen, der die Lasing-Reaktion der Biomolekül-Virus-Verbindungen störungsfrei misst;
   c) Pumpen der flüssigen Lösung, die die Biomolekül-Virus-Verbindung umfasst, mit Licht einer Wellenlänge und/oder einer Intensität, die auf die Art des wirksamen Mechanismus der Farbstoffe, vorzugsweise des wirksamen Fluoreszenzmechanismus der Fluoreszenzfarbstoffe, abgestimmt ist, mit denen das Virus markiert ist;
   d) Verstärken des von den gepumpten Farbstoffen emittierten Lichts in einem optischen Resonator in dem Versuch, eine Lasing-Reaktion von den angeregten Farbstoffen in dem optischen Resonator zu erreichen; und
   e) Messen der Intensität des verstärkten emittierten Lichts und Auswerten der Intensität des verstärkten emittierten Lichts gegen die Intensität des verstärkten emittierten Lichts aus einer bekannten Dichte an markierten Fluorescein-Farbstoffen pro Virusmolekül oder gegen eine äquivalente Konzentration an ungebundenem markiertem Virus mit der gleichen Anzahl an Farbstoffen pro Virus, um die Bindungsfähigkeit des Biomoleküls an das markierte Virus zu bestimmen.

2. Verfahren nach Anspruch 1, wobei das Virus ein M13-Bakteriophage oder ein rekombinantes Tabakmosaikvirus-ähnliches Partikel (rTMV) ist.

3. Verfahren nach einem der vorhergehenden Ansprüche, wobei das Biomolekül ein Antikörper oder ein monoklonaler Antikörper, vorzugsweise ein monoklonaler IgG2a-Antikörper (mAB) ist.

4. Verfahren nach einem der vorhergehenden Ansprüche, wobei der Fluorescein-Farbstoff ein Fluorescein-Isothiocyanat-Isomer-1-Farbstoff ist.

5. Verfahren nach einem der vorhergehenden Ansprüche, wobei das Virus mit dem Fluorescein-Farbstoff kovalent modifiziert ist.

6. Verfahren nach einem der vorhergehenden Ansprüche, wobei das Pumpen unter Verwendung von Anregungspulsen von 1 ns bis 20 ns bei einer Wellenlänge erreicht wird, die innerhalb des sichtbaren Spektrums liegt, vorzugsweise im Bereich von 450 bis 600 nm.

7. Verfahren nach einem der vorhergehenden Ansprüche, wobei die flüssige Lösung zwischen einem Reservoir und einer Durchflussküvette zirkuliert wird.

8. Verfahren nach einem der vorhergehenden Ansprüche, wobei das Virus effektiv mit einer spektralen Peak-Emission genetisch programmierbar ist, die durch Variieren der Anzahl der Fluorescein-Farbstoffe, die pro Virus angebracht sind, und/oder durch Modifizieren der chemischen Beschaffenheit der Oberfläche des Virus abstimmbar ist.

9. Verfahren nach Anspruch 8, **dadurch gekennzeichnet, dass** das Virus mit einer Anzahl von Fluorescein-Farbstoffen konjugiert ist, die im Mittel pro Ring von Hüllproteinen des Virus einem Bereich von 0,5 bis 2 Farbstoffen, vorzugsweise 0,7 bis 1,2 Farbstoffen, entsprechen.

**Revendications**

1. Procédé de biodétection d'une capacité de liaison entre une biomolécule, telle qu'un anticorps, une protéine membranaire ou similaire, et un virus, comprenant les étapes de :

a) marquage du virus par conjugaison d'une pluralité de colorants, de préférence des colorants à base de fluorescéine, au virus afin d'obtenir un virus marqué pouvant être contrôlé en ce qui concerne la densité de colorants marqués par molécule virale ;

b) liaison en phase de solutions du virus marqué à la biomolécule dans une solution liquide dans la mesure des caractéristiques de liaison entre la biomolécule et le virus marqué afin de générer des composés de biomolécule-virus et de permettre un dosage homogène de liaison au ligand par mélange et mesure qui mesure de manière non perturbatrice la réponse laser des composés de biomolécule-virus ;

c) pompage de la solution liquide comprenant le composé de biomolécule-virus avec une lumière ayant une longueur d'onde et/ou une intensité adaptée au type de mécanisme efficace des colorants, de préférence le mécanisme de fluorescence efficace des colorants à base de fluorescéine, marqués sur le virus ;

d) amplification de la lumière émise par les colorants pompés dans un résonateur optique dans le but d'obtenir une réponse laser provenant des colorants excités dans le résonateur optique ; et

e) mesure de l'intensité de la lumière émise amplifiée et évaluation de l'intensité de la lumière émise amplifiée par rapport à l'intensité de la lumière émise amplifiée à partir d'une densité connue de colorants à base de fluorescéine marqués par molécule virale ou par rapport à une concentration équivalente de virus marqué non lié avec le même nombre de colorants par virus afin de déterminer la capacité de liaison de la biomolécule au virus marqué.

2. Procédé selon la revendication 1, dans lequel le virus est un bactériophage M13 ou une particule semblable au virus de la mosaïque du tabac recombinant (rTMV).

3. Procédé selon l'une quelconque des revendications précédentes, dans lequel la biomolécule est un anticorps ou un anticorps monoclonal, de préférence un anticorps monoclonal IgG2a (mAB).

4. Procédé selon l'une quelconque des revendications précédentes, dans lequel le colorant à base de fluorescéine est un colorant isomère 1 d'isothiocyanate de fluorescéine.

5. Procédé selon l'une quelconque des revendications précédentes, dans lequel le virus est modifié par covalence avec le colorant à base de fluorescéine.

6. Procédé selon l'une quelconque des revendications précédentes, dans lequel le pompage est réalisé en utilisant des impulsions d'excitation de 1 ns à 20 ns à une longueur d'onde qui se situe dans le spectre visible, de préférence dans la plage de 450 à 600 nm.

7. Procédé selon l'une quelconque des revendications précédentes, dans lequel la solution liquide est circulée entre un réservoir et une cuvette à écoulement.

8. Procédé selon l'une quelconque des revendications précédentes, dans lequel le virus est génétiquement programmable de façon efficace avec une émission de pic spectral qui est réglable en faisant varier le nombre de colorants à base de fluorescéine fixés par virus et/ou en modifiant le paysage chimique de la surface du virus.

9. Procédé selon la revendication 8, dans lequel le virus est conjugué avec un nombre de colorants à base de fluorescéine équivalent à une plage de 0,5 à 2 colorants, de préférence de 0,7 à 1,2 colorant, en moyenne par anneau de protéines d'enveloppe du virus.

Fig. 1

EP 3 729 086 B1

Fig. 2

21

Fig. 3

**Fig. 4**

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2794861 A1

**Non-patent literature cited in the description**

- **HALES J E.** *Implementing the synthetic biology design cycle to fabricate laser dyes,* 31 December 2014, vol. 11, URL:http://iris.ucl.ac.uk/iris/publication/955819/1

- **XUDONG FAN et al.** The potential of optofluidic biolasers. *NATURE METHODS,* 01 February 2014, vol. 11 (2), ISSN 1548-7091, 141-147